# EUROPÄISCHE PATENTSCHRIFT

(11) **EP 2 174 704 B1**
(45) Veröffentlichungstag und Bekanntmachung des Hinweises auf die Patenterteilung: **01.02.2012**
(21) Anmeldenummer: 08017564.9
(22) Anmeldetag: 07.10.2008
(51) Int. Cl.: B01F 7/00

(54) **Vorrichtung zur Wartungsbereitstellung eines höhenverstellbaren, angetriebenen Tauchgerätes an einem Biogasanlagen-Fermenterbehälter**
Device for performing maintenance on a height-adjustable, powered submersible device on a biogas facility fermentation container
Dispositif de mise à disposition pour l'entretien d'un appareil de plongée entraîné et réglable en hauteur sur un récipient de fermentation d'installations de biogaz

(43) Veröffentlichungstag der Anmeldung: 14.04.2010
(73) Patentinhaber: R.E.U.S. Energy GmbH, 83527 Kirchdorf (DE)
(72) Erfinder: Chowdhury, Rajib Pal, 83512 Wasserburg (DE); Bürger, Adam, 83527 Haag/Obb. (DE)

(56) Entgegenhaltungen:
- DE-A1- 4 120 987
- DE-A1-102007 009 451
- DE-C1- 19 517 901
- DE-C1- 19 714 342
- DE-C1- 19 732 198

## Beschreibung

Die Erfindung betrifft eine Vorrichtung zur Wartungsbereitstellung eines höhenverstellbaren, angetriebenen Tauchgeräts insbesondere eines Tauchrührwerks oder einer Tauchpumpe an einem Biogasanlagen-Fermenterbehälter.

Es sind allgemein Ausführungen von Biogasanlagen-Fermenterbehältern bekannt, bei denen ein Tauchgerät an einem vertikalen, im Fermenterbehälter angebrachten Führungsmast höhenverschiebbar gehalten ist, wobei der Führungsmast mit einem Mastoberteil durch eine Deckenöffnung aus dem Fermenterbehälter nach oben herausgeführt ist. An diesem Mastoberteil ist eine Höhenverstelleinrichtung angeordnet mit einer betätigbaren Seilrolle zum auf- und abwickeln eines Zugseils, das mit dem Tauchgerät und/oder einem mit dem Tauchgerät verbundenen Führungsschlitten am Führungsmast verbunden ist. Die Deckenöffnung ist so groß ausgeführt und die Höhenverstelleinrichtung so hoch am Mastoberteil angeordnet, dass das Tauchgerät mit der Höhenverstelleinrichtung aus der Deckenöffnung des Fermenterbehälters herausziehbar ist.

Dazu ist es weiter bekannt (DE 197 14 342 C1) einen Dom über der Deckenöffnung anzubringen, in den ein Rührgerät in eine Wartungsposition verlagerbar ist. Durch eine öffenbare Domöffnung ist dann das Tauchgerät für einen Monteur zu Montage- und/oder Wartungsarbeiten zugänglich. Aus Platz- und Kostengründen wird die Größe eines solchen Doms möglichst klein gehalten, so dass die Zugänglichkeit zum Tauchgerät eingeschränkt ist und Arbeiten im Dom beengt und unbequem auszuführen sind. Dies kann ein Sicherheitsrisiko sein, weil oft auch über der offenen Deckenöffnung gearbeitet wird. Im Vergleich zu früher werden derzeit insbesondere Tauchrührgeräte mit relativ großen Rührflügeln eingesetzt, da solche Tauchrührgeräte zu einem vergleichsweise besseren Rührergebnis als Tauchrührgeräte mit kleinen Rührflügeln führen. Dadurch ergeben sich Probleme einen gesamten Rührpropeller durch die relativ kleine Domöffnung zur Wartungs- oder Austauchzwecken hindurch zu bewegen, was beispielsweise zu aufwendigen Lösungen mit zerlegbaren Rührpropellem mit demontierbaren Rührflügeln geführt hat.

Aufgabe der Erfindung ist es demgegenüber eine Vorrichtung zur Wartungsbereitstellung eines höhenverstellbaren, angetriebenen Tauchgeräts an einem Biogasanlagen-Fermenterbehälter so weiterzubilden, dass bei einfacher Handhabung und einfachem Aufbau die Zugänglichkeit zu einem Tauchgerät in der Wartungsposition und die Arbeitssicherheit verbessert werden. Diese Aufgabe wird mit den Merkmalen des Anspruches 1 gelöst.

Gemäß Anspruch 1 ist im Fermenterbetrieb und im normalen Tauchgerätebetrieb die Deckenöffnung durch eine Podestplatte sowie ein angrenzendes, die Ebene der Podestplatte überragendes Mastgehäuse gasdicht abgedeckt. Im Mastgehäuse ist der Mastoberteil einschließlich der Höhenverstelleinrichtung mit der Seilrolle, dem zugeordnetem Zugseil und gegebenenfalls einer Getriebeeinheit dergestalt eingehaust, dass das Mastgehäuse Seitenwände aufweist, nach unten zum Fermenterbehälter offen ist und oben durch eine Gehäuseabdeckung verschlossen ist, die ein Stützlager für das obere Mastende enthält.

Zumindest eine Seitenwand des Mastgehäuses ist in unmittelbarer Nähe des Mastoberteils angeordnet und als bedarfsweise öffenbare Wartungswand ausgeführt. Im unteren Bereich der öffenbaren Wartungswand grenzt die ebenfalls bedarfsweise öffenbare Podestplatte an. Für eine Wartungsbereitstellung des Tauchgeräts zur Demontage, Montage, Reparatur, Wartung, für einen Teileaustausch, etc. sind die Wartungswand und die Podestplatte öffenbar. Das Tauchgerät ist mittels der Seilrolle und dem Zugseil nach oben über die Ebene der geöffneten Podestplatte aus dem Fermenterbehälter aushebbar. Dabei ist das Tauchgerät, auch mit einem relativ großen Rührpropeller vor der geöffneten Wartungswand frei zugänglich und es bestehen keine beengten Arbeitsbedingungen. Die Podestplatte kann unter dem ausgehobenen Tauchgerät wieder einfach in ihre Abdeckposition gebracht werden, wobei gegebenenfalls verbleibende Spalte einfach gasdicht abdeckbar sind, so dass lediglich ein geringer Gasverlust während des Aushebevorgangs besteht und die Arbeitssicherheit durch austretendes Biogas nicht beeinträchtigt ist. Durch die Einhausung des Mastoberteils einschließlich der Höhenverstelleinrichtung mit der Seilrolle, dem zugeordneten Zugseitlen und gegebenenfalls einer Getriebeeinheit sind diese Teile geschützt und eine hinsichtlich der Dichtheit problematische Zugseildurchführung aus dem Gasraum nach außen ist nicht erforderlich. Zudem entsteht dadurch die Möglichkeit den Führungsmast um seine vertikal Achse schwenkbar zu gestalten, so dass das Tauchgerät in unterschiedliche Schwenkpositionen positionierbar ist.

In einer vorteilhafter konkreten Ausführungsform nach Anspruch 2 ist die Deckenöffnung etwa rechteckig gestaltet und der Führungsmast bzw. der Mastoberteil ist im mittleren Bereich einer Rechteckschmalseite angeordnet, wodurch die Größe der Deckenöffnung für den Durchtritt eines auch relativ großen Tauchgeräts gut ausgenützt ist. Dabei kann das Mastgehäuse relativ klein sein und nur im mittleren Bereich dieser Rechteckschmalseite den Mastoberteil umfassen. Alternativ kann sich das Mastgehäuse auch als schmales längliches Mastgehäuse über den ganzen Bereich dieser Rechteckschmalseite bis jeweils zu den gegenüberliegenden Rechtecklangseiten erstrecken. Dabei wird zweckmäßig das Mastgehäuse nach Anspruch 3 durch ein Rahmengestell gebildet mit einer Beplankung durch die Seitenwände, von denen wenigstens die Wartungswand öffenbar ist, und durch die Gehäuseabdeckung, wobei bedarfsweise Stützstreben unmittelbar oder mittelbar mit dem Rahmengestell verbunden sein können, um gegebenenfalls die Stabilität der Abstützung des oberen Mastendes zu verbessern.

Je nach den Gegebenheiten und Erfordernissen kann es nach Anspruch 4 zweckmäßig sein die Podestplatte und die Wartungswand zusammen einstückig oder mehrteilig auszuführen.

Dabei können nach Anspruch 5 die Podestplatte und/oder Wartungswand durch lösbare Verschraubungen und/oder Schnellverschlüsse gehalten sein und/oder im zugeordneten Winkelrahmen einliegen, sowie für eine Öffnung abnehmbar oder klappbar oder verschiebbar oder verschwenkbar ausgeführt sein.

Für eine verbesserte Handhabung wird mit Anspruch 6 vorgeschlagen, die Podestplatte und/oder die Wartungswand jeweils an der Außenseite mit Handgriffen auszurüsten, die vorzugsweise aus Griffmulden ausschwenkbar sind.

Nach Anspruch 7 eignen sich das Mastgehäuse und/oder die Podestplatte gut für die Anbringung von Sichtfenstern.

Vorteilhaft ist nach Anspruch 8 der Führungsmast in an sich bekannter Weise um seine Vertikalachse schwenkbar gelagert, wobei das Stützlager in der Gehäuseabdeckung als gasdichtes Schwenklager ausgeführt ist, durch das ein Koaxial mit dem Führungsmast verbundener Lagerzapfen gegebenenfalls für eine Kurbelbetätigung nach außen geführt ist.

In an sich bekannter Weise kann nach Anspruch 9 über die Lagerzapfendurchführung sowie gegebenenfalls über eine oder mehrere weitere Antriebsdurchführungen und Winkelgetriebe die Seilrolle für eine Höheneinstellung und der Führungsmast für eine Richtungsposition des Tauchgeräts in an sich bekannter Weise manuell eingestellt werden.

Die erfindungsgemäße Vorrichtung eignet sich nach Anspruch 10 für die Anbringung an einer horizontalen Fermenterbehälterdecke, insbesondere an einer Betondecke, wobei dann das Mastgehäuse über diese Fermenterbehälterdecke nach oben abragt.

Nach Anspruch 11 ist die erfindungsgemäße Vorrichtung auch für Fermenterbehälter mit einem geneigt verlaufenden Foliendach in der Art eines Zeltdachs gut geeignet. Dabei liegt die Deckenöffnung im Randbereich des Foliendachs zu einer Fermenterbehälterwand hin. Im Bereich der Deckenöffnung ist ein Podestgestell angebracht. Auf diesem sind horizontal die Podestplatte sowie nach oben abragend das Mastgehäuse angeordnet. Das umgebende Foliendach ist mittels Dachfolienbereichen und/oder weiteren Folienteilen und/oder mit Plattenteilen, insbesondere Blechplatten dicht angeschlossen. Das Podestgestell kann über relativ lange Steher am Fermenterbehälterboden abgestützt sein. Eine weniger aufwendige Konstruktion wird nach Anspruch 12 dadurch erhalten, dass das Podestgestell über schräg nach unten, zur Fermenterbehälterwand verlaufende Anschlussstützen befestigt ist.

Der Platz und die Zugänglichkeit für Arbeiten am ausgehobenen Tauchgerät werden vorteilhaft dadurch vergrößert, dass nach Anspruch 13 in der Ebene der Podestplatte etwa über der Fermenterbehälterwand und an dieser abgestützt eine feste Arbeitsbühne vorzugsweise mit Geländer angebracht ist.

Das Podestgestell mit der Podestplatte und dem Mastgestell kann vom geneigt verlaufenden Foliendacht vorspringen. Diese ergibt eine relativ hochbauende Konstruktion. Falls dies nicht gewünscht ist, kann das Podestgestell mit der Podestplatte einen Dacheinschnitt bilden, wodurch die Konstruktion insgesamt niedriger wird.

Anhand einer Zeichnung wird die Erfindung näher erläutert.

Es zeigen:
- Fig. 1: einen schematischen Teilausschnitt aus einem Biogasanlagen-Fermenterbehälter mit einem an einem Führungsmast höhenverstellbar gehaltenen Tauchrührgerät und einer Vorrichtung zur Wartungsbereitstellung;
- Fig. 2: eine Draufsicht auf eine weiter konkretisierte Ausführungsform gemäß Fig. 1;
- Fig. 3: einen Längsschnitt durch die Ausführungsform nach Fig. 2 mit hochgezogenem Tauchrührgerät;
- Fig.4: eine schematische Schnittdarstellung einer Seitenansicht einer weiteren Ausfüh- rungsform einer Vorrichtung mit einem aus einem schrägverlaufenden Foliendach vorspringenden Podestgestell;
- Fig. 5: eine Draufsicht auf die Ausführungsform nach Fig. 4; und
- Fig. 6: einen Schnitt einer Seitenansicht einer modifizierten Ausführungsform entsprechend Fig. 4 mit einem Podestgestell in einem Dacheinschnitt.

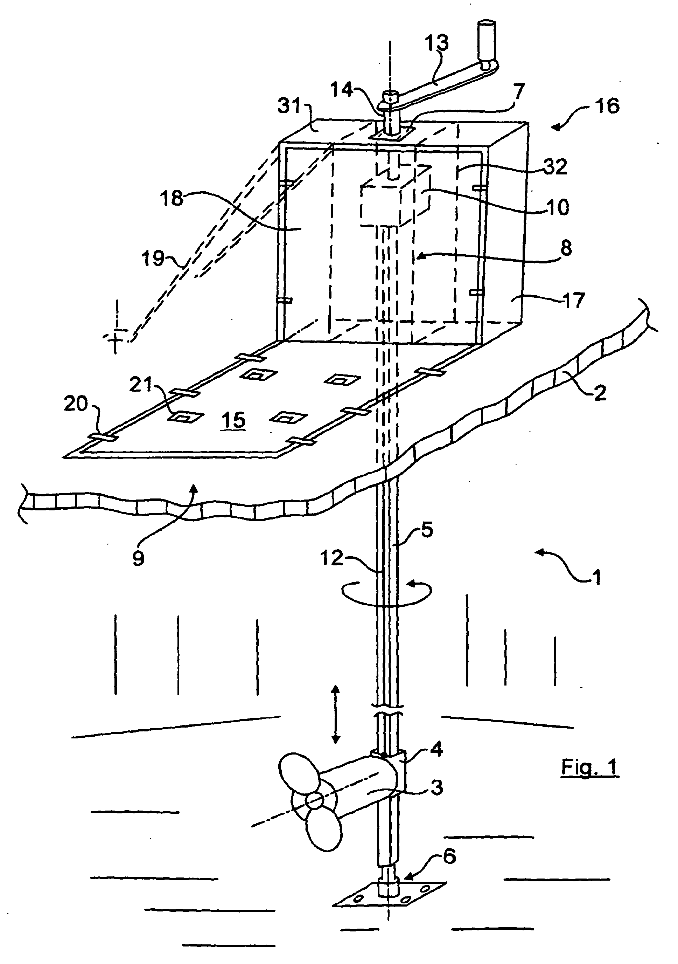

In Fig. 1 ist schematisch ein Teilausschnitt aus einem Biogasanlagen-Fermenterbehälter 1 mit einer Betondecke 2 gezeigt. Ein Tauchrührgerät 3 ist an einem Führungsschlitten 4 befestigt, der an einem Führungsmast 5 in der Art eines Quadratrohrs verschiebbar gehalten ist. Der Führungsmast 5 steht vertikal im Fermenterbehälter 1 und ist in einem unteren Lager 6 und einem oberen, als gasdichtes Schwenklager 7 ausgeführten Stützlager schwenkbar gehalten.

Der Führungsmast 5 ist mit einem Mastoberteil 8 durch eine rechteckige Deckenöffnung 9 aus dem Fermenterbehälter 1 nach oben herausgeführt.

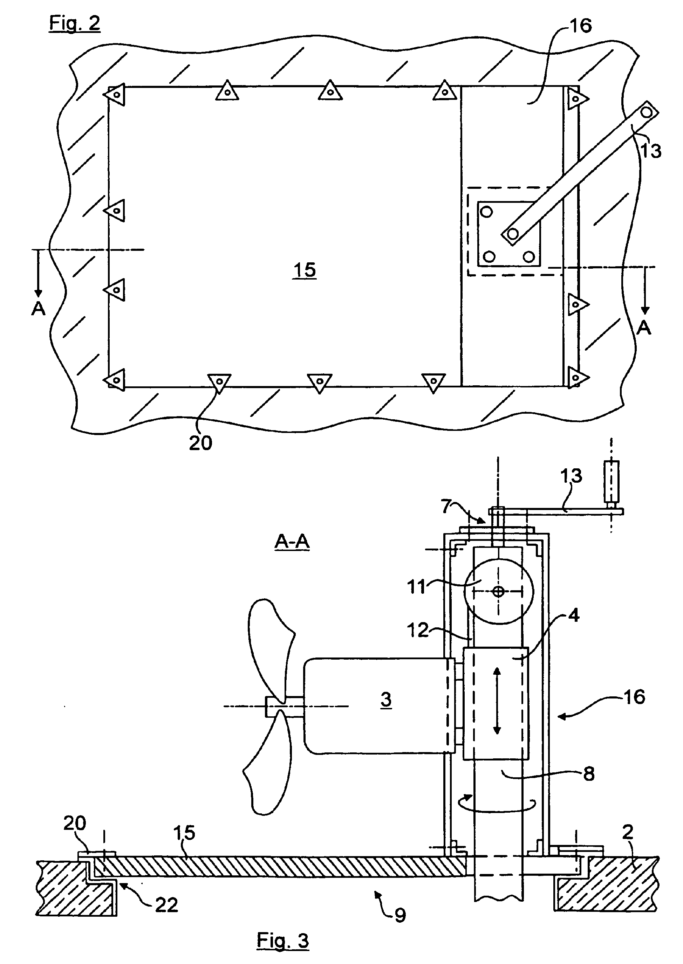

Im oberen Bereich des Mastoberteils 8 ist eine hier nur schematisch angedeutete Höhenverstelleinrichtung 10 angebracht, die wie konkret aus Fig. 3 ersichtlich, eine betätigbare Seilrolle 11 mit einem zugeordnetem Zugseil 12 aufweist, welches für eine Höhenverstellung mit dem Führungsschlitten 4 und damit mit dem Tauchrührwerk 3 verbunden ist. Die Höhenverstellung kann mit der Höhenverstelleinrichtung beispielsweise mit einer außenliegenden Kurbel 13 über einen Lagerzapfen 14 und einem der Seilrolle 11 vorgeschalteten (nicht dargestellten) Winkelgetriebe erfolgen. Alternativ kann die Seilrollenbetätigung auch gesteuert motorisch erfolgen. Die Schwenkverstellung des Führungsmasts 5 kann in an sich bekannter Weise ebenfalls von Hand beispielsweise über eine Doppelwelle im Lagerzapfen 14 erfolgen.

Die Deckenöffnung 9 ist im normalen Tauchgerätbetrieb durch eine Podestplatte 15 und durch ein angrenzendes, die Ebene der Podestplatte 15 überragendes Mastgehäuse 16 gasdicht abgedeckt. Mit dem Mastgehäuse 16 ist der Mastoberteil 8 mit der Höhenverstelleinrichtung 10 eingehaust, wobei das Mastgehäuse 16 Seitenwände 17 aufweist, nach unten zum Fermenterbehälter 1 offen ist und oben durch eine Gehäuseabdeckung 31 verschlossen ist, die das Stützlager für das obere Mastende in der Art eines gasdichten Schwenklagers 7 enthält. Der Führungsmast 5 ist mit seinem Mastoberteil 8 im mittleren Bereich einer Rechteckschmalseite der rechtwinkligen Deckenöffnung 9 angeordnet, wo auch das Mastgehäuse 16 liegt. Die zur angrenzenden Podestplatte 15 hinweisende Seitenwand des Mastgehäuses 16 ist als bedarfsweise öffenbare Wartungswand 18 ausgeführt und liegt in unmittelbarer Nähe angrenzend an das Mastoberteil 8, so dass nach einem Öffnen der Wartungswand 18 unmittelbar vor dem Mastoberteil 8 ein frei zugänglicher Raum gebildet ist.

Wie in Fig. 1 dargestellt erstreckt sich das Mastgehäuse 16 über den ganzen Bereich der Rechteckschmalseite der Deckenöffnung 9. Es reicht jedoch gegebenenfalls auch aus, das Mastgehäuse 16 mit geringerer Breite auszuführen, wie dies mit den strichlierten Linien 19 eingezeichnet ist.

Das Mastgehäuse 16 ist durch ein (nicht dargestelltes) Rahmengestell gebildet und mit den Seitenwänden 17 und der Gehäuseabdeckung 31 beplankt, wobei die Wartungswand 18 bedarfsweise öffenbar ist. Zur einer Verbesserung der Stabilität kam das Mastgehäuse 16 gegebenenfalls durch Stützstreben 19 an der Betondecke 2 abgestützt sein, von denen lediglich zwei beispielhaft strichliert eingezeichnet sind.

Die Wartungswand 18 und die Podestplatte 15 sind hier als zwei separate Bauteile ausgebildet, wobei die Podestplatte 15 das Mastgehäuse 16 zumindest teilweise auch untergreifen kann, wie aus Fig. 3 ersichtlich. Ebenso wie die Wartungswand 18 kann die Podestplatte 15 an einem Winkelrahmen 22 ausgehoben und geöffnet werden. Dazu sind hier schematisch dargestellte Schnellverschlüsse 20 zu öffnen und die Podestplatte 15 kann mittels aufklappbarer Handgriffe 21 aus dem aus Fig. 3 ersichtlichen Winkelrahmen 22 ausgehoben und entfernt werden, so dass die Deckenöffnung 9 vor dem Mastoberteil 8 freigegeben wird. Einzelheiten sind der Draufsicht nach Fig. 2 sowie der Schnittdarstellung nach Fig. 3 zu entnehmen.

In Fig. 1 ist das Tauchrührgerät 3 in der eingetauchten Funktionsstellung dargestellt. Für eine Wartungsbereitstellung werden die Wartungswand 18 und die Podestplatte 15 geöffnet und entfemet und anschließend wird das Tauchrührgerät 3 in eine obere Position über die Ebene der Podestplatte 15 heraus bewegt, anschließend wird sofort die Podestplatte 15 wieder eigesetzt und die Deckenöffnung 9 unter dem ausgehobenen Tauchrührgerät 3 wieder verschlossen, wie aus Fig. 3 ersichtlich. Gegebenenfalls verbleibende Spalte können einfach, beispielsweise mittels Lappen noch gasdicht abgedeckt werden. Damit steht das Tauchrührgerät 3 völlig frei zugänglich über der begehbaren Podestplatte 15 für Wartungs- und/oder Reparaturarbeiten und/oder eine Demontage bereit.

Um das Tauchrührgerät 3 wieder in seine Funktionsposition in den Fermenterbehälter 1 zu bringen wird umgekehrt verfahren, d. h. die Podestplatte 15 wird wieder entfernt, das Tauchrührgerät 3 abgesenkt und anschließend werden die Podestplatte 15 und die Wartungswand 18 wieder eingesetzt und gasdicht verschlossen.

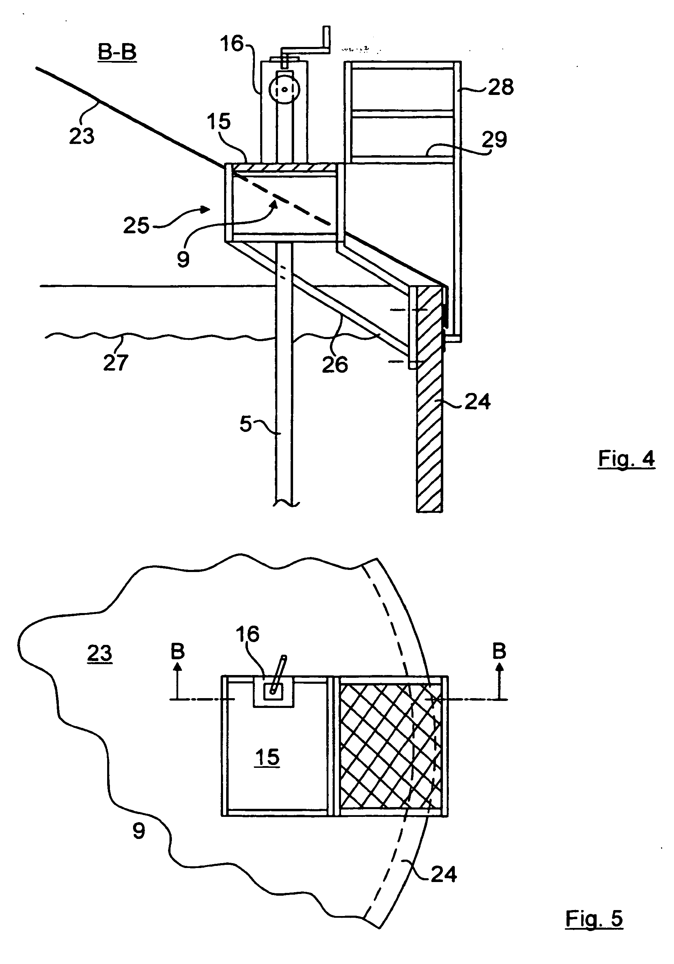

In den Fig. 4 und 5 ist in schematischer Form eine Ausführungsform der Bereitstellungseinrichtung in Verbindung mit einem geneigt verlaufenden Foliendach 23 gezeigt. Dabei liegt eine Deckenöffnung 9 im Randbereich des Foliendachs 23 in der Nähe zu einer Fermenterbehälterwand 24. Im Bereich der Deckenöffnung 9 ist ein Podestgestell 25 angebracht auf dem horizontal die Podestplatte 15 liegt, sowie nach oben abragend das Mastgehäuse 16 angeordnet ist. Das das Podestgestell 25 umgebende Foliendach 23 ist gasdicht angeschlossen, wozu Dachfolienränder oder weitere Folienteile oder Blechteile verwendbar sind. Das Podestgestell 25 ist mit schräg nach unten zur Fermenterbehätterwand 24 hin verlaufenden Anschlussstützen 26 befestigt. Mit der Wellenlinie 27 ist der maximale Fermenterfüllstand angegeben.

In der Ebene der Podestplatte 15 ist eine von einem Geländer 28 umgebene Arbeitsbühne 29 oberhalb der Fermenterbehälterwand 24 angebracht und dort abgestützt. Die Mittel und Maßnahmen für die Wartungsbereitstellung eines (hier nicht dargestellten) Tauchrührgeräts 3 mit der öffenbaren Podestplatte 15 und Wartungswand 18 entsprechen bei der Ausführung nach den Fig. 4 und 5 der Ausführung nach den Fig. 1 bis 3.

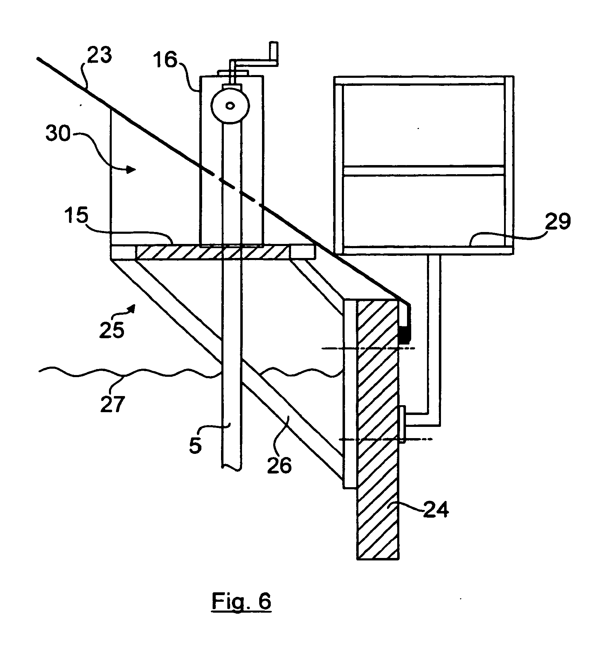

Aus Fig. 4 ist ersichtlich, dass das Podestgestell 25 mit der horizontalen Podestplatte 15 aus der Neigungsebene des Foliendachs 23 in Richtung der Fermenterbehälterwand 24 vorspringt, wodurch sich ein relativ hoher Aufbau insbesondere auch für die Arbeitsbühne 29 ergibt. Bei einer modifizierten Ausführungsform nach Fig. 6 mit im Wesentlichen gleichen Aufbau wird dagegen ein Podestgestell 25 mit der öffenbaren Podestplatte 15 und dem Mastgehäuse 16 in einem Dacheinschnitt 30 angeordnet und entsprechend abgedichtet, so dass sich damit insgesamt insbesondere auch für die Arbeitsbühne 29 eine vergleichsweise niedrigere Konstruktion ergibt.

## Patentansprüche

1. Vorrichtung zur Wartungsbereitstellung eines höhenverstellbaren, angetriebenen Tauchgeräts, insbesondere eines Tauchrührgeräts oder einer Tauchpumpe an einem Biogasanlagen-Fermenterbehälter, wobei
- das Tauchgerät (3) an einem vertikalen, im Fermenterbehälter (1) angebrachten Führungsmast (5) höhenverschiebbar gehalten ist und der Führungsmast (5) mit einem Mastoberteil (8) durch eine Deckenöffnung (9) aus dem Fermenterbehälter (1) nach oben herausgeführt ist,
- eine Höhenverstelleinrichtung (10) am Mastoberteil (8) angeordnet ist mit einer betätigbaren Seilrolle (11) zum Auf- und Abwickeln eines Zugseils (12), das mit dem Tauchgerät (3) und/oder mit einem am Führungsmast (5) verschiebbaren und am Tauchgerät (3) befestigten Führungsschlitten (4) verbunden ist, und
- die Deckenöffnung (9) so groß ist und die Höhenverstelleinrichtung (10) so hoch am Mastoberteil (8) angebracht ist, dass das Tauchgerät (3) aus der Deckenöffnung (9) des Fermenterbehätters (1) herausziehbar ist,
**dadurch gekennzeichnet,**
- **dass** im normalen Tauchgerätbetrieb die Deckenöffnung (9) durch eine Podestplatte (15) und ein angrenzendes, die Ebene der Podestplatte (15) überragendes Mastgehäuse (16) gasdicht abgedeckt ist,
- **dass** im Mastgehäuse (16) der Mastoberteil (8) einschließlich der Höhenverstelleinrichtung (10) mit der Seilrolle (11), dem zugeordneten Zugseil (12) und gegebenenfalls einer Getriebeeinheit dergestalt eingehaust ist, dass das Mastgehäuse (16) Seitenwände (17) aufweist, nach unten zum Fermenterbehälter (1) offen ist und oben durch eine Gehäuseabdeckung (31) verschlossen ist, die ein Stützlager (7) für das obere Mastende enthält,
- **dass** zumindest eine Seitenwand (17) des Mastgehäuses (16) in unmittelbarer Nähe des Mastoberteils (8) angeordnet ist und als bedarfsweise öffenbare Wartungswand (18) ausgeführt ist,
- **dass** im unteren Bereich der öffenbaren Wartungswand (18) die ebenfalls bedarfsweise öffenbare Podestplatte (15) angrenzt, so dass für eine Wartungsbereitstellung des Tauchgeräts (3), die Wartungswand (18) und die Podestplatte (15) öffenbar sind und das Tauchgerät (3) mittels der Seilrolle (11) und dem Zugseil (12) nach oben über die Ebene der geöffneten Podestplatte (15) aus dem Fermenterbehälter (1) aushebbar ist, wobei das Tauchgerät (3) im Bereich vor der geöffneten Wartungswand (18) frei zugänglich ist und anschließend die Podestplatte (15) unter dem ausgehobenen Tauchgerät (3) wieder anbringbar und gegebenenfalls verbleibende Spalte gasdicht abdeckbar sind.

2. Vorrichtung nach Anspruch 1, **dadurch gekennzeichnet, dass** die Deckenöffnung (9) etwa rechteckig ist und der Führungsmast (5) bzw. der Mastoberteil (8) im mittleren Bereich einer Rechteckschmalseite angeordnet ist, und
dass das Mastgehäuse (16) nur im mittleren Bereich (strichlierte Linien 32) dieser Rechteckschmalseite angeordnet ist oder sich als schmales Mastgehäuse (16) über den ganzen Bereich dieser Rechteckschmalseite bis jeweils zu den gegenüberliegenden Rechtecklangseiten erstreckt.

3. Vorrichtung nach Anspruch 1 oder 2, **dadurch gekennzeichnet, dass** das Mastgehäuse (16) durch ein Rahmengestell gebildet ist mit einer Beplankung durch die Seitenwände (17), von denen wenigstens die Wartungswand (18) öffenbar ist, und durch die Gehäuseabdeckung (31), wobei bedarfsweise Stützstreben (19) unmittelbar oder mittelbar mit dem Rahmengestell verbindbar sind.

4. Vorrichtung nach einem der Ansprüche 1 bis 3, **dadurch gekennzeichnet, dass** die Podestplatte (15) und die Wartungswand (18) zusammen einstückig oder mehrteilig ausgeführt sind.

5. Vorrichtung nach einem der Ansprüche 1 bis 4, **dadurch gekennzeichnet, dass** die Podestplatte (15) und/oder die Wartungswand (18) durch lösbare Verschraubungen und/oder Schnellverschlüsse (20) gehalten sind und/oder in zugeordneten Winkelrahmen (22) einliegen, sowie für eine Öffnung abnehmbar oder klappbar oder verschiebbar oder verschwenkbar ausgeführt sind.

6. Vorrichtung nach einem der Ansprüche 1 bis 4, **dadurch gekennzeichnet, dass** die Podestplatte (15) und/oder die Wartungswand (18) jeweils an der Außenseite mit Handgriffen (21), vorzugsweise mit aus Griffmulden (21) einschwenkbaren Handgriffen ausgerüstet sind.

7. Vorrichtung nach einem der Ansprüche 1 bis 6, **dadurch gekennzeichnet, dass** im Mastgehäuse (16) und/oder in der Podestplatte (15) Sichtfenster angebracht sind.

8. Vorrichtung nach einem der Ansprüche 1 bis 7, **dadurch gekennzeichnet, dass** der Führungsmast (5) um seine Vertikalachse schwenkbar gelagert ist und das Stützlager in der Gehäuseabdeckung (31) als gasdichtes Schwenklager (7) ausgeführt ist, durch das ein koaxial mit dem Führungsmast (5) verbundener Lagerzapfen (14) gegebenenfalls für eine Betätigung mit einer Kurbel (13) nach außen geführt ist.

9. Vorrichtung nach Anspruch 8, **dadurch gekennzeichnet, dass** über die Lagerzapfendurchführung und gegebenenfalls über eine oder mehrere weitere Antriebsdurchführungen sowohl die Seilrolle (11), gegebenenfalls über ein Winkelgetriebe, für eine Höheneinstellung des Tauchgeräts (3) betätigbar ist, als auch die Schwenkstellung des Führungsmasts (5) für eine Richtungsposition des Tauchgeräts (3) einstellbar ist.

10. Vorrichtung nach einem der Ansprüche 1 bis 9, **dadurch gekennzeichnet, dass** die Deckenöffnung (9) in einer horizontalen Fermenterbehälterdecke (2), insbesondere einer Betondecke liegt und das Mastgehäuse (16) über die Fermenterbehälterdecke (2) nach oben abragt.

11. Vorrichtung nach einem der Ansprüche 1 bis 9, **dadurch gekennzeichnet, dass** die Deckenöffnung (9) in einem geneigt verlaufenden Foliendach (23) in dessen Randbereich zu einer Fermenterbehälterwand (24) liegt,
dass im Bereich der Deckenöffnung (9) ein Podestgestell (25) angebracht ist, auf dem horizontal die Podestplatte (15) sowie nach oben abragend das Mastgehäuse (16) angeordnet sind und das umgebende Foliendach (23) mittels Dachfolienbereichen und/oder weiteren Folienteilen und/oder Plattenteilen dicht angeschlossen ist.

12. Vorrichtung nach Anspruch 11, **dadurch gekennzeichnet, dass** das Podestgestell (25) schräg nach unten zur Fermenterbehälterwand (24) verlaufende Anschlussstützen (26) aufweist.

13. Vorrichtung nach Anspruch 11 oder 12, **dadurch gekennzeichnet, dass** in der Ebene der Podestplatte (15) über der Fermenterbehälterwand (24) und an dieser abgestützt eine Arbeitsbühne (29), vorzugsweise mit Geländer (28) angebracht ist.

14. Vorrichtung nach einem der Ansprüche 11 bis 13, **dadurch gekennzeichnet, dass** das Podestgestell (25) mit der Podestplatte (15) und dem Mastgehäuse (16) vom geneigt verlaufenden Foliendach (23) vorspringen oder einen Dacheinschnitt (30) bilden.

## Claims

1. An apparatus for providing maintenance on a height-adjustable, driven submersible appliance, in particular a submersible stirrer or a submersible pump on a fermenter of a biogas plant, wherein
- the submersible appliance (3) is held and can be displaced in height on a vertical guide mast (5) positioned in the fermenter (1) and a top part (8) of the guide mast (5) is directed upwardly out of the fermenter (1) through a ceiling opening (9),
- a height-adjusting device (10) is arranged on the top part (8) of the mast and has an actuable cable pulley (11) for winding up and unwinding a traction cable (12) which is connected to the submersible appliance (3) and/or to a guide carriage (4) which can be displaced on the guide mast (5) and is fixed to the submersible appliance (3), and
- the ceiling opening (9) is so large and the height-adjusting device (10) is positioned so high on the top part (8) of the mast that the submersible appliance (3) can be pulled out of the ceiling opening (9) of the fermenter (1),
**characterized**
- **in that**, in normal operation of the submersible appliance, the ceiling opening (9) is covered in a gas-tight manner by a platform panel (15) and an adjoining mast housing (16), which projects above the plane of the platform panel (15),
- **in that** the top part (8) of the mast, including the height-adjusting device (10) having the cable pulley (11), the associated traction cable (12) and optionally a gear-mechanism unit, is encased in the mast housing (16) such that the mast housing (16) has side walls (17), is open at the bottom toward the fermenter (1) and is closed at the top by a housing cover (31) which contains a supporting bearing (7) for the upper end of the mast,
- **in that** at least one side wall (17) of the mast housing (16) is arranged in the immediate vicinity of the top part (8) of the mast and is configured as a maintenance wall (18) which can be opened as required,
- **in that** in the bottom region the openable maintenance wall (18) adjoins the platform panel (15), which can likewise be opened as required, so that, in order to provide maintenance on the submersible appliance (3), the maintenance wall (18) and the platform panel (15) can be opened and the submersible appliance (3) can be lifted out of the fermenter (1), upwardly beyond the plane of the opened platform panel (15) by means of the cable pulley (11) and the traction cable (12), wherein the submersible appliance (3) is freely accessible in the region in front of the opened maintenance wall (18) and subsequently the platform panel (15) can be positioned under the lifted-out submersible appliance (3) again and possibly remaining gaps can be covered in a gas-tight manner.

2. The apparatus according to claim 1, **characterized in that** the ceiling opening (9) is approximately rectangular and the guide mast (5), or the top part (8) of the mast, is arranged in the central region of a narrow side of the rectangle, and
**in that** the mast housing (16) is arranged only in the central region (dashed lines 32) of this narrow side of the rectangle or extends as a narrow mast housing (16) over the entire region of this narrow side of the rectangle as far as the opposing long sides of the rectangle, respectively.

3. The apparatus according to claim 1 or 2, **characterized in that** the mast housing (16) is formed by a framework panelled by the side walls (17), of which at least the maintenance wall (18) can be opened, and by the housing cover (31), wherein supporting struts (19) can be connected as required directly or indirectly to the framework.

4. The apparatus according to any one of claims 1 to 3, **characterized in that** the platform panel (15) and the maintenance wall (18) are configured together in one piece or in multiple parts.

5. The apparatus according to any one of claims 1 to 4, **characterized in that** the platform panel (15) and/or the maintenance wall (18) is/are held by releasable screw connections and/or quick-release fasteners (20) and/or is/are located in associated angle frames (22), and is/are configured such that it/they can be removed or swung or displaced or pivoted for opening purposes.

6. The apparatus according to any one of claims 1 to 4, **characterized in that** the platform panel (15) and/or the maintenance wall (18), respectively, is/are equipped on the outside with handles (21), preferably with handles that can be pivoted out of handle recesses (21).

7. The apparatus according to any one of claims 1 to 6, **characterized in that** viewing windows are provided in the mast housing (16) and/or in the platform panel (15).

8. The apparatus according to any one of claims 1 to 7, **characterized in that** the guide mast (5) is mounted such that it can be pivoted about its vertical axis, and that the supporting bearing in the housing cover (31) is configured as a gas-tight pivot bearing (7), through which a bearing journal (14), which is connected coaxially to the guide mast (5), is guided outwards optionally for being actuated by means of a crank (13).

9. The apparatus according to claim 8, **characterized in that**, via the bearing journal bushing and optionally via one or more further drive bushings, both the cable pulley (11) can be actuated, optionally via an angular gear mechanism, in order to adjust the height of the submersible appliance (3), and the pivot position of the guide mast (5) can be adjusted for setting the direction of the submersible appliance (3).

10. The apparatus according to any one of claims 1 to 9, **characterized in that** the ceiling opening (9) is located in a horizontal fermenter ceiling (2), in particular a concrete ceiling, and that the mast housing (16) projects upwardly beyond the fermenter ceiling (2).

11. The apparatus according to any one of claims 1 to 9, **characterized in that** the ceiling opening (9) is located in an inclined film roof (23), in the peripheral region thereof to a fermenter wall (24),
**in that**, in the region of the ceiling opening (9) there is installed a platform framework (25), on which the platform panel (15) is arranged horizontally and the mast housing (16) is arranged such that it projects upwards, and the surrounding film roof (23) is connected in a sealed manner by means of roof film regions and/or further film parts and/or panel parts.

12. The apparatus according to claim 11, **characterized in that** the platform framework (25) has connection struts (26) that extend obliquely downward to the fermenter wall (24).

13. The apparatus according to claim 11 or 12, **characterized in that** a working platform (29), preferably having a railing (28), is arranged in the plane of the platform panel (15), above and supported on the fermenter wall (24).

14. The apparatus according to any one of claims 11 to 13, **characterized in that** the platform framework (25) with the platform panel (15) and the mast housing (16) projects from the inclined film roof (23) or forms a roof incision (30).

## Revendications

1. Un dispositif pour mettre à disposition l'entretien d'un appareil immergé entraîné et réglable en hauteur, en particulier d'un appareil agitateur immergé ou d'une pompe immergée sur un récipient de fermentation d'installations de biogaz, dans lequel
- l'appareil (3) immergé est maintenu de façon réglable en hauteur sur un mât (5) de guidage installé dans le récipient (1) de fermentation et le mât (5) de guidage est conduit vers le haut à l'extérieur du récipient (1) de fermentation avec une partie (8) supérieure de mât , à travers une ouverture (9) de plafond,
- un dispositif (10) de réglage de hauteur est disposé sur la partie (8) supérieure de mât, avec une bobine (11) de câble pouvant être actionnée pour enrouler et dérouler un câble (12) de traction, qui est relié à l'appareil (3) immergé et/ou à un chariot (4) de guidage déplaçable sur le mât (5) de guidage et fixé sur l'appareil (3) immergé, et
- l'ouverture (9) de plafond est à ce point grande et le dispositif (10) de réglage de hauteur est installé à ce point haut sur la partie (8) supérieure de mât que l'appareil (3) immergé puisse être retiré hors de l'ouverture (9) de plafond du récipient (1) de fermentation,
**caractérisé en ce que**
- en fonctionnement normal de l'appareil immergé, l'ouverture (9) de plafond est recouverte hermétiquement aux gaz par une plaque (15) de plate-forme et une enceinte (16) de mât adjacente qui dépasse le plan de la plaque (15) de plate-forme,
- la partie (8) supérieure de mât, y compris le dispositif (10) de réglage de hauteur avec la bobine (11) de câble, le câble (12) de traction associé et éventuellement une unité de transmission, est logée dans l'enceinte (16) de mât, de telle manière que l'enceinte (16) de mât présente des parois (17) latérales, et ouverte vers le bas en direction du récipient (1) de fermentation et fermée vers le haut par un couvercle (31) d'enceinte, qui contient un palier (7) d'appui pour l'extrémité supérieure du mât,
- au moins une paroi (17) latérale de l'enceinte (16) de mât est disposée à proximité immédiate de la partie (8) supérieure de mât et est réalisée comme une paroi (18) d'entretien pouvant être ouverte en cas de nécessité,
- la plaque (15) de plate-forme pouvant également être ouverte en cas de nécessité est adjacente, dans la région inférieure, à la paroi (18) d'entretien pouvant être ouverte, de telle manière que, pour mettre à disposition un entretien de l'appareil (3) immergé, la paroi (18) d'entretien et la plaque (15) de plate-forme puissent être ouvertes et que l'appareil (3) immergé puisse être hissé vers le haut, hors du récipient (1) de fermentation au-dessus du plan de la plaque (15) de plate-forme ouverte, au moyen de la bobine (11) de câble et du câble (12) de traction, dans lequel l'appareil (3) immergé est alors librement accessible dans la région devant la paroi (18) d'entretien ouverte et la plaque (15) de plate-forme peut ensuite être de nouveau placée sous l'appareil (3) immergé hissé et des fentes subsistant peuvent éventuellement être recouvertes hermétiquement aux gaz..

2. Le dispositif selon la revendication 1, **caractérisé en ce que** l'ouverture (9) de plafond est sensiblement rectangulaire et le mât (5) de guidage ou la partie (8) supérieure de mât est disposée dans la région centrale d'un côté étroit du rectangle, et **en ce que** l'enceinte (16) de mât n'est disposée que dans la région centrale de ce côté étroit du rectangle (traits 32 interrompus) ou s'étend comme étroite enceinte (16) de mât sur toute la région de ce côté étroit du rectangle respectivement jusqu'aux longs côtés opposés du rectangle.

3. Le dispositif selon la revendication 1 ou 2, **caractérisé en ce que** l'enceinte (16) de mât est formée par une structure de cadre avec une fermeture par les parois (17) latérales, parmi lesquelles au moins la paroi (18) d'entretien peut être ouverte, et par le couvercle (31) d'enceinte, dans lequel des entretoises (19) de soutien peuvent au besoin être reliées directement ou indirectement à la structure de cadre.

4. Le dispositif selon l'une quelconque des revendications 1 à 3, **caractérisé en ce que** la plaque (15) de plate-forme et la paroi (18) d'entretien sont réalisées ensemble d'une seule pièce ou en plusieurs pièces.

5. Le dispositif selon l'une quelconque des revendications 1 à 4, **caractérisé en ce que** la plaque (15) de plate-forme et/ou la paroi (18) d'entretien sont maintenues par des vis démontables et/ou des raccords (20) rapides et/ou sont posées dans des cadres (22) angulaires associés, et sont réalisées en vue d'une ouverture par enlèvement ou relèvement ou glissement ou pivotement.

6. Le dispositif selon l'une quelconque des revendications 1 à 4, **caractérisé en ce que** la plaque (15) de plate-forme et/ou la paroi (18) d'entretien sont respectivement équipées sur le côté extérieur avec des poignées, de préférence des poignées pivotantes hors de coquilles (21) de poignée.

7. Le dispositif selon l'une quelconque des revendications 1 à 6, **caractérisé en ce que** des hublots d'observation sont placés dans l'enceinte (16) de mât et/ou dans la plaque (15) de plate-forme.

8. Le dispositif selon l'une quelconque des revendications 1 à 7, **caractérisé en ce que** le mât (5) de guidage est monté de façon pivotante autour de son axe vertical et le palier d'appui dans le couvercle (31) d'enceinte est réalisé sous la forme d'un palier (7) pivotant hermétique aux gaz, à travers lequel un tourillon (14) assemblé de façon coaxiale au mât (5) de guidage est conduit éventuellement vers l'extérieur en vue d'un actionnement avec une manivelle (13).

9. Le dispositif selon la revendication 8, **caractérisé en ce qu'**aussi bien la bobine (11) de câble, le cas échéant au moyen d'un engrenage angulaire, peut être actionnée pour un réglage en hauteur de l'appareil (3) immergé, que la position de pivotement du mât (5) de guidage peut être réglée pour une position de la direction de l'appareil (3) immergé, au moyen de la partie sortante du tourillon et éventuellement aussi par une ou plusieurs autres parties sortantes d'entraînement.

10. Le dispositif selon l'une quelconque des revendications 1 à 9, **caractérisé en ce que** l'ouverture (9) de plafond est située dans un plafond (2) horizontal du récipient de fermentation, en particulier un plafond de béton, et l'enceinte (16) de mât est saillante vers le haut au-dessus du plafond (2) du récipient de fermentation.

11. Le dispositif selon l'une quelconque des revendications 1 à 9, **caractérisé en ce que** l'ouverture (9) de plafond est située dans un toit (23) incliné en film, dans la région du bord de celui-ci vers une paroi (24) du récipient de fermentation,
et **en ce que**, dans la région de l'ouverture (9) de plafond est installée une structure (25) de plate-forme, sur laquelle la plaque (15) de plate-forme est installée horizontalement ainsi que l'enceinte (16) de mât saillante vers le haut, et le toit (23) en film environnant est raccordé hermétiquement au moyen de régions de feuilles de toit et/ou d'autres parties de feuille et/ou de parties de plaques.

12. Le dispositif selon la revendication 11, **caractérisé en ce que** la structure (25) de plate-forme présente des appuis (26) de raccordement s'étendant en oblique vers le bas vers la paroi (24) du récipient de fermentation.

13. Le dispositif selon la revendication 11 ou 12, **caractérisé en ce qu'**une plate-forme (29) de travail de préférence avec une balustrade (28), est installée dans le plan de la plaque (15) de plate-forme au-dessus de la paroi (24) du récipient de fermentation et s'appuie sur celle-ci.

14. Le dispositif selon l'une quelconque des revendications 11 à 13, **caractérisé en ce que** la structure (25) de plate-forme avec la plaque (15) de plate-forme et l'enceinte (16) de mât est saillante sur le toit (23) incliné en film ou forme une découpe (30) dans le toit.
